(19) Europäisches Patentamt
European Patent Office
Office européen des brevets

(11) Veröffentlichungsnummer: **0 019 690**
**B1**

(12) # EUROPÄISCHE PATENTSCHRIFT

(45) Veröffentlichungstag der Patentschrift: **21.04.82**

(51) Int. Cl.³: **C 07 J 53/00,** A 61 K 31/565, A 61 K 31/585

(21) Anmeldenummer: **80101383.0**

(22) Anmeldetag: **17.03.80**

(54) **6 beta.7 beta; 15.16-Dimethylen-1.4-androstadien-3-one, Verfahren zu ihrer Herstellung und Verwendung als Arzneimittel.**

(30) Priorität: **31.05.79 DE 2922500**

(43) Veröffentlichungstag der Anmeldung: **10.12.80 Patentblatt 80/25**

(45) Bekanntmachung des Hinweises auf die Patenterteilung: **21.04.82 Patentblatt 82/16**

(84) Benannte Vertragsstaaten: **AT BE CH DE FR GB IT LU NL SE**

(56) Entgegenhaltungen:
**DE-A-1 914 507**
**DE-A-2 235 646**
**DE-A-2 264 189**
**DE-A-2 652 761**
**FR-A-2 391 224**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Wiechert, Rudolf, Prof. Dr., Petzowerstrasse 8A, D-1000 Berlin 37 (DE)**
Erfinder: **Bittler, Dieter, Bölkauer Pfad 11, D-1000 Berlin 27 (DE)**
Erfinder: **Kerb, Ulrich, Dr., Prinzregentenstrasse 7, D-1000 Berlin 31 (DE)**
Erfinder: **Prezewowsky, Klaus, Dr., Aarauer Strasse 10, D-1000 Berlin 45 (DE)**
Erfinder: **Casals-Stenzel, Jorge, Dr., Wichernstrasse 20, D-1000 Berlin 31 (DE)**
Erfinder: **Losert, Wolfgang, Prof. Dr., Niedstrasse 12, D-1000 Berlin 41 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

6 beta.7 beta; 15.16-Dimethylen-1.4-androstadien-3-one, Verfahren zu ihrer Herstellung und Verwendung als Arzneimittel

Die Erfindung betrifft neue 6β.7β; 15.16-Dimethylen-1.4-androstadien-3-one der allgemeinen Formel

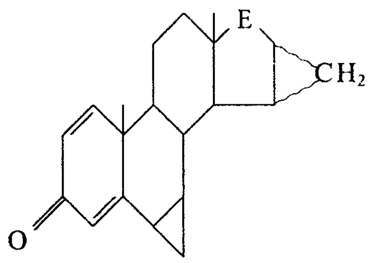

worin die 15.16-ständige Methylengruppe α- oder β-ständig sein kann und

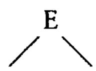

für die Gruppierung

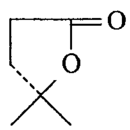

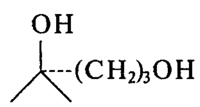

oder

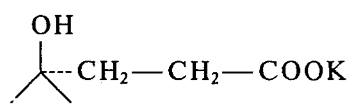

steht.

Aus der deutschen Offenlegungsschrift 2 652 761 und der französischen Patentschrift 2 391 224 sind bereits Spironolactone bzw. 17β-Hydroxy-17α-(3-hydroxypropyl)-4-androsten-3-one mit einer 6,7-Methylengruppierung bekannt. Die beschriebenen Verbindungen können als Aldosteron-Antagonisten verwendet werden. Die erfindungsgemäßen Verbindungen besitzen eine $\Delta^1$-Doppelbindung im A-Ring des Steroidmoleküls, währenddessen die bekannten Verbindungen in 1.2-Stellung gesättigt sind.

Die erfindungsgemäßen Verbindungen besitzen wertvolle pharmakologische Eigenschaften. Sie sind u. a. Diuretika vom Typ der Aldosteron-Antagonisten, d. h., sie kehren die Wirkung von Desoxycorticosteron auf die Natrium- und Kaliumausscheidung um. Die erfindungsgemäßen Verbindungen erweisen sich im Testmodell von Hollmann (G. Hollmann et al., Tubuläre Wirkungen und renale Elimination von Spirolactonen, Naunyn-Schmiedebergs Arch. Exp. Path. Pharmak. 247 (1964) 419; P. Marx, Renale Wirkungen des d-Aldosterons und seines Antagonisten Spironolactons, Diss. Med. Fak. FU Berlin 1966) dem bekannten Spironolacton in ihrer Wirkung überraschenderweise überlegen.

Die Erfindung betrifft weiterhin ein Verfahren zur Herstellung von 6β.7β; 15.16-Dimethylen-1.4-androstadien-3-onen der allgemeinen Formel

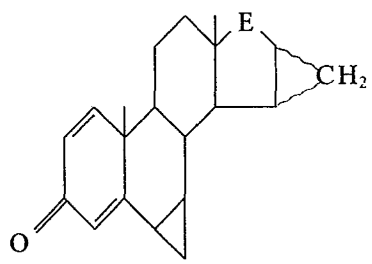

worin die 15.16-ständige Methylengruppe α- oder β-ständig sein kann und

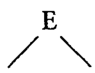

für die Gruppierung

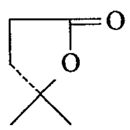

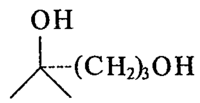

oder

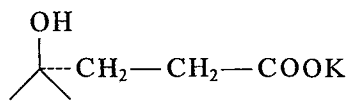

steht, welches dadurch gekennzeichnet ist, daß man in an sich bekannter Weise in entsprechende 6β.17β;15.16-Dimethylen-4-androsten-3-one die $\Delta^1$-Doppelbindung einführt und gegebenenfalls den Lactonring anschließend aufspaltet.

Die Einführung der $\Delta^1$-Doppelbindung erfolgt nach an sich bekannten Methoden und kann auf chemischem oder mikrobiologischem Wege erfolgen. Geeignete chemische Dehydrierungsmittel zur 1.2-Dehydrierung sind beispielsweise Selendioxid, 2.3-Dichlor-5.6-dicyanobenzochinon, Chloranil, Thalliumtriacetat oder Bleitetraacetat. Geeignete Mikroorganismen für die 1.2-Dehydrierung sind beispielsweise Schizomyceten, insbesondere solche der Genera Arthrobacter, wie z. B. simplex ATCC 6946; Bacillus, wie z. B. B. lentus ATCC 13 805 und B. sphaericus ATCC 7055; Pseudomonas, wie z. B. P. aeruginosa IFO 3505; Flavobacterium, wie z. B. F. flavescens IFO 3058; Lactobacillus, wie z. B. L. brevis IFO 3345 und Nocardia, wie z. B. N. opaca ATCC 4276.

Die 1.2-Dehydrierung wird bevorzugt chemisch ausgeführt. Hierzu wird das 1.2-Dihydrosteroid in einem geeigneten Lösungsmittel mit dem Dehydrierungsmittel über längere Zeit erwärmt. Geeignete Lösungsmittel sind beispielsweise Dioxan, tert. Butanol, Tetrahydrofuran, Toluol, Benzol bzw. Gemische dieser Lösungsmittel.

Die Reaktion ist nach mehreren Stunden abgeschlossen. Es empfiehlt sich, die Umsetzung durch Dünnschichtchromatographie zu verfolgen. Das Reaktionsgemisch wird aufgearbeitet, wenn das Ausgangsmaterial umgesetzt ist.

Die sich gegebenenfalls anschließende Öffnung des Lactonringes erfolgt ebenfalls nach an sich bekannten Methoden. Hierzu wird das Lacton mit verdünnter Kalilauge erwärmt, wobei sich das Kaliumsalz der 3-substituierten Propionsäure bildet.

Nach beendeter Reaktion wird das Reaktionsgemisch in üblicher Weise wie Fällung, Extraktion, Umkristallisation und/oder Chromatographie aufgearbeitet.

Die erfindungsgemäßen Verbindungen werden nach an sich bekannten Methoden der Galenik zur Herstellung von Arzneimitteln für die orale und parenterale Applikation verwendet.

Die Dosierung der erfindungsgemäßen Verbindung liegt beim Menschen bei 10–200 mg/Tag.

Die nachfolgenden Beispiele sollen die Erfindung erläutern.

Beispiel 1

500 mg 6β.7β; 15β.16β-Dimethylen-4-androsten-[17(β-1′)-spiro-5′]perhydrofuran-2′.3-dion werden in 7,5 ml Dioxan mit 500 mg 2.3-Dichlor-5.6-dicyan-p-benzochinon 17 Stunden am Rückfluß erhitzt. Es wird dann mit Äther verdünnt, mit gesättigter Natriumhydrogencarbonatlösung und Wasser gewaschen, getrocknet und eingedampft. Der Rückstand wird an Silicagel chromatographiert. Es werden 330 mg 6β.7β; 15β.16β-Dimethylen-1.4-androsta-dien-[17(β-1′)-spiro-5′]perhydrofuran-2′.3-dion vom Schmelzpunkt 250,5 – 252° C erhalten.
UV: $\varepsilon_{245} = 12\,000$, $\varepsilon_{286} = 9950$.

Beispiel 2

300 mg 6β.7β; 15α.16α-Dimethylen-4-androsten-[17(β-1′)-spiro-5′]perhydrofuran-2′.3-dion werden in 15 ml tert.-Butanol mit 90 mg Selendioxid und 0,5 ml Essigsäure 20 Stunden am Rückfluß gerührt. Es werden dann nochmals 90 mg Selendioxid nachgegeben und weitere 24 Stunden am Rückfluß erhitzt. Nach dem Eindampfen zur Trockne wird der Rückstand an Silicagel chromatographiert. Es werden 120 mg 6β.7β; 15α.16α-Dimethylen-1.4-androstadien-[17(β-1′)-spiro-5′]perhydrofuran-2′.3-dion erhalten.
UV: $\varepsilon_{245} = 11\,800$, $\varepsilon_{285} = 9800$.

Beispiel 3

500 mg 6β.7β; 15β.16β-Dimethylen-1.4-androstadien-[17(β-1′)-spiro-5′]perhydrofuran-2′.3-dion werden in 5 ml Isopropanol mit 1,37 ml 1 n Kaliumhydroxid in Methanol versetzt und 1 Stunde am Rückfluß erhitzt. Nach dem Abkühlen werden 30 ml absoluter Äther zugesetzt und 1 Stunde im Eisbad nachgerührt. Es wird dann der auskristallisierte Niederschlag abgesaugt, mit Äther gut nachgewaschen und getrocknet. Man erhält 510 mg 3(17β-Hydroxy-6β.7β; 15β.16β-dimethylen-3-oxo-1.4-androstadien-17α-yl)-propionsäure-Kaliumsalz.
UV: $\varepsilon_{245} = 11\,500$; $\varepsilon_{286} = 9600$.

Beispiel 4

300 mg 17β-Hydroxy-17α-(3-hydroxypropyl)-6β.7β; 15β.16β-dimethylen-4-androsten-3-on werden in 9 ml Dioxan mit 300 mg 2.3-Dichlor-5.6-dicyan-p-benzochinon 17 Stunden am Rückfluß erhitzt. Es wird dann mit Äther verdünnt, mit gesättigter Natriumhydrogencarbonatlösung und Wasser gewaschen, getrocknet und eingedampft. Der Rückstand wird an Silicagel chromatographiert. Es werden als amorphe Substanz 120 mg 17β-Hydroxy-17α-(3-hydroxypropyl)-6β.7β; 15β.16β-dimethylen-1.4-androstadien-3-on erhalten.
UV: $\varepsilon_{244} = 11\,500$; $\varepsilon_{285} = 9600$.

**Patentansprüche**

1. Verfahren zur Herstellung von 6β.7β; 15.16-Dimethylen-1.4-androstadien-3-onen der allgemeinen Formel

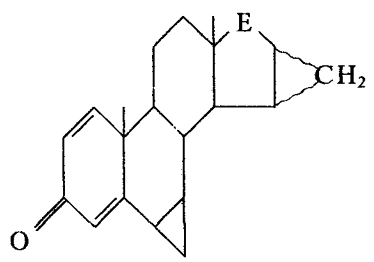

worin die 15.16-ständige Methylengruppe α- oder β-ständig sein kann und

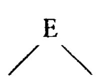

für die Gruppierung

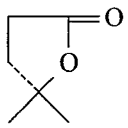

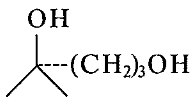

oder

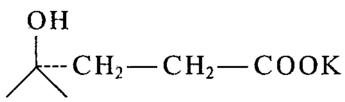

steht, dadurch gekennzeichnet, daß man in an sich bekannter Weise in entsprechende 6β.7β;15.16-Dimethylen-4-androsten-3-one die $\Delta^1$-Doppelbindung einführt und gegebenenfalls den Lactonring anschließend aufspaltet.

2. Verfahren nach Anspruch 1, dadurch gekennzeichnet, daß man die 1.2-Dehydrierung mit Selendioxid ausführt.

3. Verfahren nach Anspruch 1, dadurch gekennzeichnet, daß man die 1.2-Dehydrierung mit 2.3-Dichlor-5.6-dicyanobenzochinon ausführt.

4. 6β.7β; 15.16-Dimethylen-1.4-androstadien-3-one der allgemeinen Formel

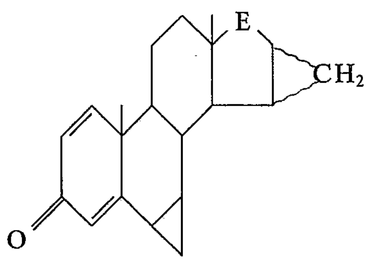

worin die 15.16-ständige Methylengruppe α- oder β-ständig sein kann und

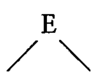

für die Gruppierung

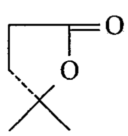

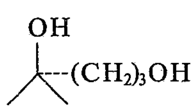

oder

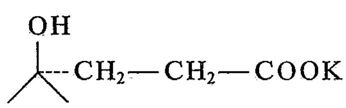

steht.

5. 6β.7β; 15β.16β-Dimethylen-1.4-androstadien-[17(β-1′)-spiro-5′]perhydrofuran-2′.3-dion.

6. 6β.7β; 15α.16α-Dimethylen-1.4-androstadien-[17(β-1′)-spiro-5′]perhydrofuran-2′.3-dion.

7. 3(17β-Hydroxy-6β.7β;15β.16β-dimethylen-3-oxo-1.4-androstadien-17α-yl)-propionsäure-Kalium-salz.

8. 17$\beta$-Hydroxy-17$\alpha$-(3-hydroxypropyl)-6$\beta$.7$\beta$; 15$\beta$.16$\beta$-dimethylen-1.4-androstadien-3-on.
9. Arzneimittel auf Basis der Verbindungen gemäß den Ansprüchen 4—8.

**Claims**

1. A process for the preparation of a 6$\beta$,7$\beta$;15,16-dimethylene-1,4-androstadien-3-one of the general formula

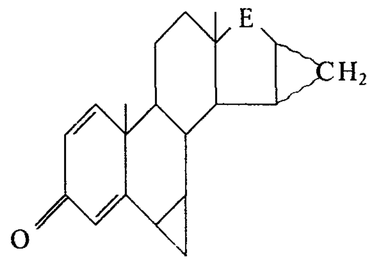

(I)

in which the 15,16-positioned methylene group may be in the α- or β-configuration and E represents a group of the formula

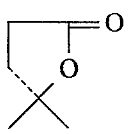

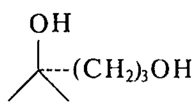

or

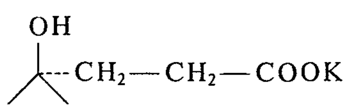

which comprises introducing the $\Delta^1$-double bond into the corresponding 6$\beta$,7$\beta$;15,16-dimethylene-4-androsten-3-one, and when required splitting the lactone ring.

2. A process as claimed in claim 1 wherein the introduction of the $\Delta^1$-double bond is carried out with selenium dioxide.

3. A process as claimed in claim 1 wherein the introduction of the $\Delta^1$-double bond is carried out with 2,3-dichloro-5,6-dicyanobenzoquinone.

4. A 6$\beta$,7$\beta$;15,16-dimethylene-1,4-androstadien-3-one of the general formula

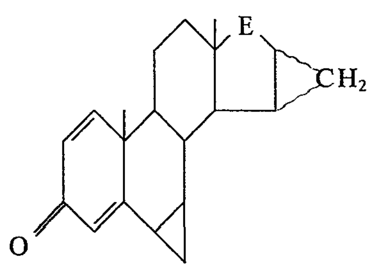

(I)

in which the 15,16-positioned methylene group may be in the α- or β-configuration and E represents a group of the formula

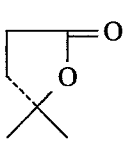

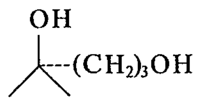

or

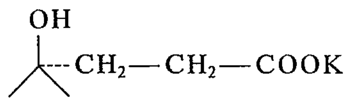

5. 6β,7β;15β,16β-Dimethylene-1,4-androstadiene-[17(β-1')spiro-5']perhydrofuran-2',3-dione.
6. 6β,7β;15α,16α-Dimethylene-1,4-androstadiene-[17(β-1')-spiro-5']perhydrofuran-2',3-dione.
7. Potassium 3-(17β-hydroxy-6β,7β;15β,16β-dimethylene-3-oxo-1,4-androstadien-17-yl)-propionate.
8. 17β-Hydroxy-17α-(3-hydroxypropyl)-6β,7β;15β,16β-dimethylene-1,4-androstadien-3-one.
9. Medicaments characterised in that they contain a compound according to claims 4 to 8.

**Revendications**

1. Procédé de préparation de diméthylène-6β,7β; 15,16 androstadiène-1,4 ones-3 répondant à la formule générale:

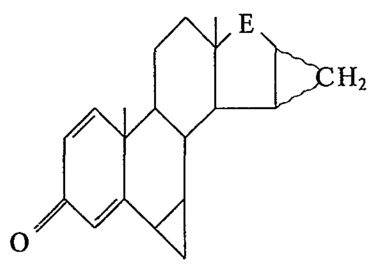

dans laquelle le radical méthylène en 15,16 peut avoir la configuration α ou la configuration β et

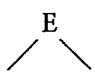

désigne l'un des groupements suivants:

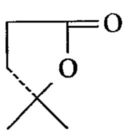

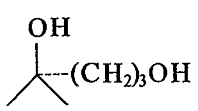

et

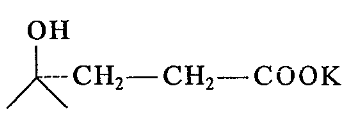

procédé caractérisé en ce qu'on introduit la double liaison $\Delta^1$ dans des diméthylène-6β,7β; 15,16 androstène-4 ones-3 correspondantes, puis on ouvre éventuellement le noyau lactone, cela par des méthodes connues.

2. Procédé selon la revendication 1, caractérisé en ce qu'on effectue la déshydrogénation en 1,2 au moyen du dioxyde de sélénium.

3. Procédé selon la revendication 1, caractérisé en ce qu'on effectue la déshydrogénation en 1,2 au moyen de la dichloro-2,3 dicyano-5,6 benzoquinone.

4. Diméthylène-6β,7β; 15,16 androstadiène-1,4 ones-3 qui répondent à la formule générale:

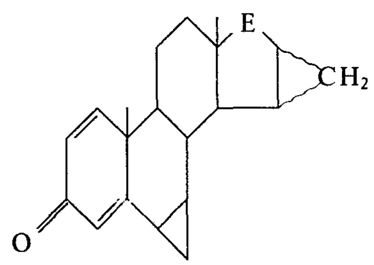

dans laquelle le radical méthylène en 15,16 peut avoir la configuration α ou la configuration β et

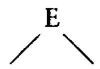

désigne l'un des groupements suivants:

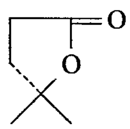

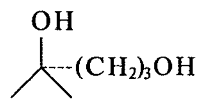

et

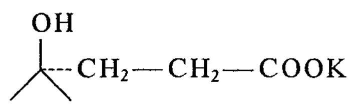

5. Diméthylène-6β,7β; 15β,16β androstadiène-1,4 [17(β-1')-spiro-5']-perhydrofuranne-dione-2',3.

6. Diméthylène 6β,7β; 15α,16α androstadiène-1,4 [17(β-1')-spiro-5']-perhydrofuranne-dione-2',3.

7. (Hydroxy-17β diméthylène-6β,7β; 15β,16β oxo-3 androstadiène-1,4 yl-17α)-3 propionate de potassium.

8. Hydroxy-17β (hydroxy-3 propyl)-17α diméthylène-6β,7β; 15β,16β androstadiène-1,4 one-3.

9. Médicament contenant un ou plusieurs des composés selon l'une quelconque des revendications 4 à 8.